# EUROPÄISCHE PATENTSCHRIFT

(11) **EP 0 400 430 B1**
(45) Veröffentlichungstag und Bekanntmachung des Hinweises auf die Patenterteilung: **17.08.1994**
(21) Anmeldenummer: 90109529.9
(22) Anmeldetag: 19.05.1990
(51) Int. Cl.: C07D 405/12, A61K 31/44

(54) **Chromanderivate**
Chroman derivatives
Dérivés de chromane

(30) Priorität: 02.06.1989 DE 3918041
(43) Veröffentlichungstag der Anmeldung: 05.12.1990
(73) Patentinhaber: MERCK PATENT GmbH, D-64271 Darmstadt (DE)
(72) Erfinder: Gericke, Rolf, Dr., D-6104 Seeheim (DE); Baumgarth, Manfred, Dr., D-6100 Darmstadt (DE); Lues, Ingeborg, Dr., D-6100 Darmstadt (DE); De Peyer, Jacques, Dr., CH-3007 Bern (CH); Bergmann, Rolf, Dr., D-6101 Reichelsheim (DE)

(56) Entgegenhaltungen:
- EP-A- 0 250 077
- EP-A- 0 273 262
- EP-A- 0 298 452
- EP-A- 0 340 718
- GB-A- 2 204 868

## Beschreibung

Die Erfindung betrifft neue Chromanderivate der Formel I 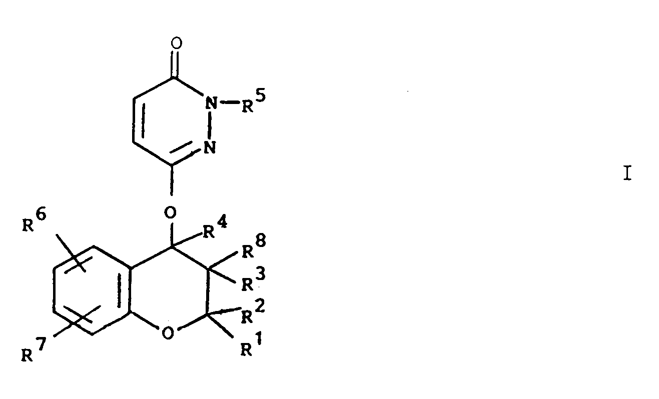
worin
- R¹: A,
- R² und R⁸: jeweils H oder A,
- R¹ und R²: zusammen auch Alkylen mit 3-6 C-Atomen,
- R³: OH, OA oder OAc,
- R⁴: H,
- R³ und R⁴: zusammen auch eine Bindung,
- R⁵: Ar oder CₙH₂ₙ-R⁹,
- R⁹: Alkenyl oder Alkinyl mit jeweils 2-4 C-Atomen, OH, OA, CHO, CO-A, CS-A, COOH, COOA, COO-alkyl-Ar, CS-OA, NO₂, NH₂, NHA, NA₂, CN, F, Cl, Br, J, CF₃, SA, SO-A, SO₂-A oder Ar,
- Ar: eine unsubstituierte oder eine ein- oder zweifach durch R¹⁰ substituierte Phenylgruppe,
- R⁶, R⁷ und R¹⁰: jeweils H, A, HO, AO, CHO, ACO, CF₃CO, ACS, HOOC, AOOC, AO-CS, ACOO, A-CS-O, Hydroxy-alkyl, Mercapto-alkyl, NO₂, NH₂, NHA, NA₂, CN, F, Cl, Br, J, CF₃, AS, ASO, ASO₂, AO-SO, AO-SO₂, AcNH, AO-CO-NH, H₂NSO, HANSO, A₂NSO, H₂NSO₂, HANSO₂ A₂NSO₂, H₂NCO, HANCO, A₂NCO, H₂NCS, HANCS, A₂NCS, ASONH, ASO₂NH, AOSONH, AOSO₂NH, ACO-alkyl, Nitro-alkyl, Cyan-alkyl, A-C(=NOH), A-C(=NNH₂), H₂PO₃ oder A₂PO₃,
- n: 1, 2 oder 3,
- A: Alkyl mit 1-6 C-Atomen,
- -alkyl: Alkylen mit 1-6 C-Atomen und
- Ac: Alkanoyl mit 1-8 C-Atomen oder Aroyl mit 7-11 C-Atomen
bedeuten,
sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z.B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z.B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls R¹ und R² zusammen Alkylen bedeuten, so ist die Alkylengruppe vorzugsweise unverzweigt, im einzelnen bevorzugt -(CH₂)ₙ-, wobei n 3, 4, 5 oder 6 bedeutet.

Die Gruppe "-alkyl" steht vorzugsweise für -CH₂- oder -CH₂CH₂-.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

R¹ und R² sind vorzugsweise jeweils Alkyl, insbesondere jeweils Methyl oder Ethyl, bevorzugt jeweils Methyl, weiterhin sind R¹ und R² zusammen bevorzugt -(CH₂)₄-oder -(CH₂)₅-.

Falls R⁴ H bedeutet, ist R³ bevorzugt OH, ferner bevorzugt O-CHO oder O-COCH₃.

Ar ist vorzugsweise unsubstituiertes Phenyl. Falls Ar eine substituierte Phenylgruppe bedeutet, so ist diese vorzugsweise einfach substituiert.

Der Parameter n ist vorzugsweise 1 oder 2.

Alkenyl ist vorzugsweise Vinyl, ferner bevorzugt 1-Propenyl oder Allyl.

In R⁶, R⁷ und R¹⁰ bedeuten vorzugsweise:
- A:: Methyl, ferner Ethyl;
- AO:: Methoxy, ferner Ethoxy;
- ACO:: Acetyl, ferner Propionyl;
- ACS:: Thioacetyl, ferner Thiopropionyl;
- AOOC:: Methoxycarbonyl, ferner Ethoxycarbonyl;
- AO-CS:: Methoxy-thiocarbonyl, ferner Ethoxythiocarbonyl;
- ACOO:: Acetoxy, ferner Propionoxy;
- ACSO:: Thio(no)acetoxy, ferner Thio(no)propionoxy;
- Hydroxyalkyl:: Hydroxymethyl oder 1- oder 2-Hydroxyethyl;
- Mercaptoalkyl:: Mercaptomethyl oder 1- oder 2-Mercaptoethyl;
- NHA:: Methylamino, ferner Ethylamino;
- NA₂:: Dimethylamino, ferner Diethylamino;
- ASO:: Methylsulfinyl, ferner Ethylsulfinyl;
- ASO₂:: Methylsulfonyl, ferner Ethylsulfonyl;
- AO-SO:: Methoxy-sulfinyl, ferner Ethoxy-sulfinyl;
- AO-SO₂:: Methoxy-sulfonyl, ferner Ethoxy-sulfonyl;
- Ac-NH:: Acetamido, ferner Formamido, Propionamido oder Benzamido;
- AO-CO-NH:: Methoxycarbonylamino, ferner Ethoxycarbonylamino;
- HANSO:: Methylaminosulfinyl, ferner Ethylaminosulfinyl
- A₂NSO:: Dimethylaminosulfinyl, ferner Diethylaminosulfinyl;
- HANSO₂:: Methylaminosulfonyl, ferner Ethylaminosulfonyl;
- A₂NSO₂:: Dimethylaminosulfonyl, ferner Diethylaminosulfonyl;
- HANCO:: N-Methylcarbamoyl, ferner N-Ethylcarbamoyl;
- A₂NOC:: N,N-Dimethylcarbamoyl, ferner N,N-Diethylcarbamoyl;
- HANCS:: N-Methyl-thiocarbamoyl, ferner N-Ethylthiocarbamoyl;
- A₂NCS:: N,N-Dimethyl-thiocarbamoyl, ferner N,N-Diethyl-thiocarbamoyl;
- ASONH:: Methylsulfinylamino, ferner Ethylsulfinylamino;
- ASO₂NH:: Methylsulfonylamino, ferner Ethylsulfonylamino;
- AOSONH:: Methoxysulfinylamino, ferner Ethoxysulfinylamino;
- AOSO₂NH:: Methoxysulfonylamino, ferner Ethoxysulfonylamino;
- ACO-alkyl:: 2-Oxopropyl, 2-Oxobutyl, 3-Oxobutyl, 3-Oxopentyl;
- Nitroalkyl:: Nitromethyl, 1- oder 2-Nitroethyl;
- Cyanalkyl:: Cyanmethyl, 1- oder 2-Cyanethyl;
- A-C(= NOH):: 1-Oximinoethyl, ferner 1-Oximinopropyl;
- A-C(= NNH₂):: 1-Hydrazonoethyl, ferner 1-Hydrazonopropyl.

Die Reste R⁶ und R⁷ stehen vorzugsweise in 6- und 7-Stellung des Chromansystems. Sie können jedoch auch in 5- und 6-, 5- und 7-, 5- und 8-, 6- und 8- sowie 7- und 8-Stellung stehen.

Von den Resten R⁶ und R⁷ ist vorzugsweise der eine H, während der andere von H verschieden ist. Dieser andere Rest steht vorzugsweise in 6-Stellung, aber auch in 5-, 7- oder 8-Stellung, und ist vorzugsweise CN oder NO₂, ferner bevorzugt CHO, ACO (insbesondere Acetyl), CF₃CO, AOOC (insbesondere Methoxycarbonyl oder Ethoxycarbonyl), ACOO (insbesondere Acetoxy), weiterhin bevorzugt F, Cl, Br, J, CF₃, H₂NCO, H₂NCS oder NH₂.

Der Rest R⁸ ist vorzugsweise H, weiterhin bevorzugt Methyl oder Ethyl.

R⁹ ist bevorzugt Vinyl, Ethinyl, OH, OA (insbesondere Methoxy oder Ethoxy), COOH, COOA (insbesondere Methoxycarbonyl oder Ethoxycarbonyl), COOCH₂C₆H₅, NH₂, CN oder Phenyl.

Dementsprechend ist R⁵ bevorzugt Phenyl, Allyl, Propargyl, 2-Hydroxyethyl, 2-Methoxyethyl, 2-Ethoxyethyl, Carboxymethyl, Methoxycarbonylmethyl, Ethoxycarbonylmethyl, Benzyloxycarbonylmethyl, 2-Aminoethyl, Cyanmethyl, 2-Cyanethyl, Benzyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ii ausgedrückt werden, die der Formel I entsprechen und worin die nicht näher bezeichneten Reste die bei der Formel I angegebene Bedeutung haben, worin jedoch
in Ia
   - R¹ und R²: jeweils A bedeuten;
in Ib
   - R¹ und R²: jeweils CH₃ bedeuten;
in Ic
   - R¹ und R²: zusammen Alkylen mit 3-6 C-Atomen bedeuten;
in Id
   - R⁵: Phenyl, Allyl, Propargyll 2-Hydroxyethyl, 2-AO-ethyl, Carboxymethyl, AO-CO-CH₂, Benzyloxycarbonylmethyl, 2-Aminoethyl, Cyanmethyl, 2-Cyanethyl oder Benzyl bedeutet;
in Ie
   - R⁵: Phenyl, Allyl, Propargyl, 2-Hydroxyethyl, Carboxymethyl Methoxycarbonylmethyl, Benzyloxycarbonylmethyl, 2-Aminoethyl, Cyanmethyl oder Benzyl bedeutet;
in If
   - R⁵: Allyl bedeutet;
in Ig
   - R¹ und R²: jeweils CH₃ oder zusammen -(CH₂)₄-oder -(CH₂)₅-,
   - R⁵: Phenyl, Allyl, Propargyl, 2-Hydroxyethyl, 2-AO-ethyl, Carboxymethyl, AO-CO-CH₂, Benzyloxycarbonylmethyl, 2-Aminoethyl, Cyanmethyl, 2-Cyanethyl oder Benzyl und
   - R⁸: H oder CH₃ bedeuten;
in Ih
   - R¹ und R²: jeweils CH₃,
   - R⁵: Phenyl, Allyl, Propargyl, 2-Hydroxyethyl, Carboxymethyl, Methoxycarbonylmethyl, Benzyloxycarbonylmethyl, 2-Aminoethyl, Cyanmethyl oder Benzyl und
   - R⁸: H bedeuten;
in Ii
   - R¹ und R²: jeweils CH₃,
   - R⁵: Allyl und
   - R⁸: H bedeuten.

Weiterhin sind bevorzugt Verbindungen der Formeln I′ sowie Ia′ bis Ii′, die den Formeln I sowie Ia bis Ii entsprechen, worin jedoch jeweils zusätzlich R³ H, OH, OCHO oder OCOCH₃ und R⁴ H bedeuten, insbesondere solche Verbindungen der Formeln I′ sowie Ia′ bis Ii′, worin jeweils zusätzlich R³ OH und R⁴ H bedeuten.

Weiterhin sind bevorzugt Verbindungen der Formeln I˝ sowie Ia˝ bis Ii˝, die den Formeln I sowie Ia bis Ii entsprechen, worin jedoch jeweils zusätzlich R³ und R⁴ zusammen eine Bindung bedeuten.

Ferner sind bevorzugt Verbindungen der Formeln I, I′, I˝, Ia bis Ii, Ia′ bis Ii′ sowie Ia˝ bis Ii˝, worin jeweils zusätzlich
(a)
   - R⁶: von H verschieden ist und
   - R⁷: H bedeutet;
(b)
   - R⁶: von H verschieden ist und in 6-Stellung steht und
   - R⁷: H bedeutet;
(c)
   - R⁶: NO₂, CN, CHO, ACO, HOOC, AOOC, ACOO, F, Cl, Br, J, CF₃, H₂NCO, H₂NCS oder NH₂ und
   - R⁷: H bedeutet;
(d)
   - R⁶: NO₂, CN, CHO, ACO, HOOC, AOOC, ACOO, F, Cl, Br, J, CF₃, H₂NCO, H₂NCS oder NH₂ bedeutet und in 6-Stellung steht und
   - R⁷: H bedeutet;
(e)
   - R⁶: NO₂, CN, Br oder CH₃OOC und
   - R⁷: H bedeutet;
(f)
   - R⁶: NO₂, CN, Br oder CH₃OOC bedeutet und in 6-Stellung steht und
   - R⁷: H bedeutet;
(g)
   - R⁶: NO₂ oder CN und
   - R⁷: H bedeutet;
(h)
   - R⁶: NO₂ oder CN bedeutet und in 6-Stellung steht und
   - R⁷: H bedeutet;
(i)
   - R⁶: CN und
   - R⁷: H bedeutet;
(j)
   - R⁶: CN bedeutet und in 6-Stellung steht und
   - R⁷: H bedeutet.

Im übrigen haben vor- und nachstehend die Reste R¹ bis R⁸, A, "alkyl" und Ac die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I gemäß Anspruch 1, dadurch gekennzeichnet, daß man ein Chroman der Formel II 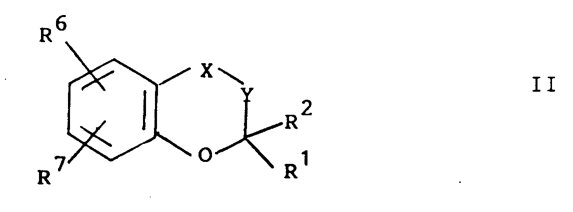
worin
- X-Y: oder -CHE-CR³R⁸- und 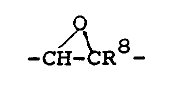
- E: Cl, Br, J oder eine reaktionsfähig veresterte OH-Gruppe bedeuten und
R¹, R², R³, R⁶, R⁷ und R⁸ die bei Formel I angegebenen Bedeutungen haben,
mit einer Verbindung der Formel III 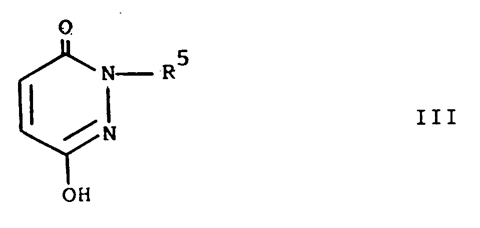
worin R⁵ die bei Formel I angegebene Bedeutung hat
oder mit einem ihrer reaktionsfähigen Derivate umsetzt
oder daß man eine Verbindung der Formel IV 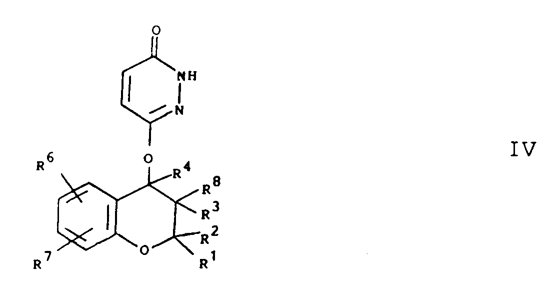
worin R¹, R², R³, R⁴, R⁶, R⁷ und R⁸ die bei Formel I angegebenen Bedeutungen haben,
oder eines ihrer reaktionsfähigen Derivate
mit einer Verbindung der Formel

E-R⁵ V

worin R⁵ und E die bei Formel I bzw. bei Formel II angegebenen Bedeutungen haben
oder einen ihrer reaktionsfähigen Derivate
umsetzt
und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶ und/oder R⁷ in andere Reste R³, R⁵, R⁶ und/oder R⁷ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150 °.

Ausgangsstoffe der Formel II mit 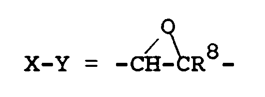
(3,4-Epoxy-chromane) sind bevorzugt.

Die Ausgangsstoffe II und III sind in der Regel bekannt (vgl. z.B. DE-OS 37 26 261). Sofern sie nicht bekannt sind, können sie nach an sich bekannten Methoden hergestellt werden. So sind die Ausgangsstoffe der Formel II 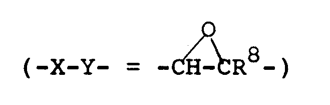
erhältlich durch Umsetzung von 2-Hydroxyacetophenonen der Formel 2-HO-R⁶R⁷C₆H₂-COCH₃ mit Ketonen der Formel R¹-CO-R² zu entsprechenden 4-Chromanonen der Formel VIa 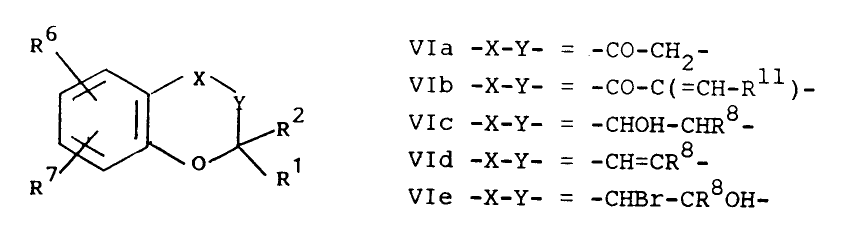
gegebenenfalls Kondensation mit Aldehyden der Formel R¹¹-CHO (R¹¹ = Alkyl mit 1-5 C-Atomen) zu 3-Alkyliden-4-chromanonen der Formel VIb, Reduktion, z.B. mit NaBH₄, zu Chromanolen der Formel VIc, Dehydratisierung, z.B. mit p-Toluolsulfonsäure, zu Chromenen der Formel VId und Oxydation, z.B. mit 3-Chlorperbenzoesäure. Die letztgenannte Oxydation kann auch mehrstufig erfolgen. So kann man, z.B. mit N-Bromsuccinimid in wäßriger Lösung, zunächst die Bromhydrine der Formel VIe herstellen und aus diesen anschließend mit einer Base, z.B. Natronlauge, HBr abspalten.

Man kann die Chromene der Formel VId auch erhalten durch Kondensation von Salicylaldehyden der Formel 2-HO-R⁶R⁷-C₆H₂-CHO mit Ketonen der Formel R¹-CO-CH₂-R⁸ zu Hydroxyketonen der Formel 2-HO-R⁶R⁷C₆H₂-CH=CR⁸-CO-R¹, Umsetzung mit Organo-Li-Verbindungen der Formel R²-Li,nachfolgende Hydrolyse zu Diolen der Formel 2-HO-R⁶R⁷C₆H₂-CH=CR⁸-CR¹R²-OH und Cyclisierung unter Wasserabspaltung.

In Verbindungen der Formeln II (-X-Y- = -CHE-CR³R⁸-) und V kommen als "reaktionsfähig veresterte OH-Gruppen" insbesondere die Ester mit Alkylsulfonsäuren (worin die Alkylgruppe 1-6 C-Atome enthält) oder mit Arylsulfonsäuren (worin die Arylgruppe 6-10 C-Atome enthält) in Betracht. Diese Verbindungen sind erhältlich aus den 4-Chromanolen der Formel VIc bzw. aus Verbindungen der Formel R⁵-OH durch Umsetzung mit einem anorganischen Säurehalogenid, wie PCl₃, PBr₃, SOCl₂ oder SOBr₂, oder mit einem Sulfonsäurechlorid, wie Methan- oder p-Toluolsulfonsäurechlorid.

Als reaktionsfähige Derivate von III eignen sich die entsprechenden Salze, z.B. die Na- oder K-Salze, die auch in situ entstehen können.

Bei der Umsetzung von II mit III ist es zweckmäßig, in Gegenwart einer Base zu arbeiten. Als Basen eignen sich z.B. Alkalimetall- oder Erdalkalimetall-hydroxide, -carbonate, -alkoholate, -hydride oder auch -amide wie NaOH, KOH, Ca(OH)₂, Na₂CO₃, K₂CO₃, Na- oder K-methylat, -ethylat oder tert.-butylat, NaH, KH, CaH₂, NaNH₂, KNH₂, ferner organische Basen wie Triethylamin oder Pyridin, die auch im Überschuß angewendet werden können und dann gleichzeitig als Lösungsmittel dienen. Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II 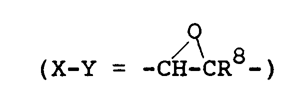
kann auch in situ hergestellt werden, z.B. durch Einwirkung einer Base auf das entsprechende Bromhydrin VIe.

Eine besonders bevorzugte Arbeitsweise besteht darin, daß man einen Alkohol (z.B. Ethanol) als Lösungsmittel verwendet und eine organische Base (z.B. Pyridin) hinzusetzt, wobei man zweckmäßig etwa 0,5 bis 20 Std. kocht.

Verbindungen der Formel I können auch hergestellt werden, indem man eine Verbindung der Formel IV mit einer Verbindung der Formel V umsetzt, zweckmäßig unter den Bedingungen einer N-Arylierung oder N-Alkylierung in Gegenwart oder Abwesenheit eines der genannten inerten Lösungsmittel (z.B. Aceton) bei Temperaturen zwischen etwa 0° und etwa 150°, wobei man vorteilhaft in Gegenwart einer der genannten Basen (z.B. Kaliumcarbonat) arbeitet.

Die Herstellung der Ausgangsstoffe der Formel IV ist z.B. in der EP-A-0273262 beschrieben; sie gelingt leicht z.B. durch Reaktion einer Verbindung der Formel II mit 3-Hydroxy-1,6-dihydro-6-pyridazinon (= 3,6-Pyridazindiol; Formel III, aber H an Stelle von R⁵). Die Ausgangsstoffe der Formel V sind in der Regel bekannt.

An Stelle der Ausgangsstoffe der Formeln IV oder V können auch ihre reaktionsfähigen Derivate eingesetzt werden, z.B. an Stelle von IV die entsprechenden K- oder Na-Salze, an Stelle von 2-Amino- oder 2-Hydroxy-1-E-alkenen der Formel V [R⁵ = -CH₂-CH(NH₂)-Cₙ₋₂H₂ₙ₋₄ bzw. -CH₂-CHOH-Cₙ₋₂H₂ₙ₋₄] die entsprechenden Alkylenimine (Aziridin, 2-Methylaziridin) bzw. die entsprechenden Alkylenoxide (Ethylenoxid, Propylenoxid); in den letztgenannten Fällen kann es zweckmäßig sein, unter erhöhtem Druck (bis zu etwa 100 bar) zu arbeiten.

Eine Verbindung der Formel I, worin R³ = OH und R⁴ = H ist, kann durch Behandeln mit einem Dehydratisierungsmittel in eine Verbindung der Formel I, worin R³ und R⁴ zusammen eine Bindung bedeuten, umgewandelt werden. Das gelingt z.B. durch Einwirkung einer der angegebenen Basen, z.B. NaH, in einem der angegebenen Lösungsmittel, z.B. DMSO, bei Temperaturen zwischen 0 und 150°.

Die Dehydratisierung kann auch mehrstufig erfolgen, indem man z.B. das Carbinol I (R³ = OH, R⁴ = H) in einen Ester, z.B. einen Sulfonsäureester wie einen Camphersulfonsäureester, umwandelt und diesen mit einer Base, z.B. mit NaOH, behandelt.

Weiterhin kann man in einer Verbindung der Formel I einen oder mehrere der Reste R³, R⁵, R⁶ und/oder R⁷ in andere Reste R³, R⁵, R⁶ und/oder R⁷ umwandeln.

Beispielsweise ist es möglich, daß man ein H-Atom mittels einer Halogenierung durch ein Halogenatom oder mittels einer Nitrierung durch eine Nitrogruppe ersetzt und/oder eine Estergruppe zu einer Carboxylgruppe verseift und/oder eine Benzyloxycarbonylgruppe zu einer Carboxylgruppe hydrogenolysiert (z.B. an Pd-C in Methanol) und/oder eine Carboxylgruppe verestert und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z.B. mit Raney-Nickel in Wasser/Essigsäure/Pyridin in Gegenwart von Natriumphosphat) in eine Formylgruppe oder (z.B. mit KOH in tert.-Butanol) in eine Carbamoylgruppe oder (z.B. mit H₂S in Pyridin/Triethylamin) in eine Thiocarbamoylgruppe umwandelt.

Eine Nitrierung gelingt unter üblichen Bedingungen, z.B. mit einem Gemisch aus konzentrierter HNO₃ und konzentrierter H₂SO₄ bei Temperaturen zwischen 0 und 30°.

Eine Halogenierung kann z.B. mit elementarem Chlor oder Brom in einem der üblichen inerten Lösungsmittel bei Temperaturen zwischen etwa 0 und 30 ° durchgeführt werden.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z.B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z.B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungsmittel, z.B. DMF, bei Temperaturen zwischen etwa 0 ° und etwa 120 ° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z.B. Chloride oder Bromide) oder Anhydride von Carbonsäuren der Formel Ac-OH, z.B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z.B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0° und etwa 160°, vorzugsweise zwischen 20° und 120°. Eine Formylierung gelingt auch mit Ameisensäure in Gegenwart von Pyridin bzw. mit einem Gemisch von Ameisensäure und Acetanhydrid.

Eine Base der Formel I kann mit einer Säure in das zugehörige Säureadditionssalz übergeführt werden. Für diese Umsetzung kommen insbesondere Säuren in Frage, die phyiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z.B. Verbindungen der Formel I, worin R¹ = R², R³ = OH und R⁴ = H ist, zwei chirale Zentren; bei der Herstellung durch Reaktion von II mit III entsteht jedoch ganz überwiegend nur ein Racemat mit trans-Stellung der OH-Gruppe in 3-Stellung und der 1-R⁵-1,6-dihydro-6-oxo-pyridazinyl-3-oxy-Gruppe in 4-Stellung. Erhaltene Racemate können, falls erwünscht, nach an sich bekannten Methoden mechanisch, chemisch oder biochemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z.B. optisch aktive Säuren, wie die D- und L-Formen von Weinsäure, Dibenzoylweinsäure, Diacetylweinsäure, Camphansäure, Camphersulfonsäuren, Mandelsäure, Äpfelsäure oder Milchsäure. Carbinole (I, R³ = OH) können ferner mit Hilfe chiraler Acylierungsreagenzien, z.B. D- oder L-α-Methylbenzylisocyanat, verestert und dann getrennt werden (vgl. EP-A1-120428). Die verschiedenen Formen der Diastereomeren können in an sich bekannter Weise, z.B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z. B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern, z. B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Verbindungen der Formel I und ihre Salze eignen sich, insbesondere bei topischer Anwendung, ferner zur Behandlung der Alopecia areata. Hierfür werden insbesondere pharmazeutische Zubereitungen verwendet, die zur topischen Behandlung der Kopfhaut geeignet und die oben genannt sind. Sie enthalten etwa 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":
Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

### Beispiel 1

Ein Gemisch von 1 g 2,2-Dimethyl-3,4-epoxy-6-cyan-chroman ("IIa"), 1,5 g 3-Hydroxy-1-phenyl-1,6-dihydro-pyridazin-6-on, 0,4 ml Pyridin und 35 ml Ethanol wird 14 Std. gekocht. Man dampft ein, chromatographiert den Rückstand an Kieselgel und erhält 2,2-Dimethyl-4-(1-phenyl-1,6-dihydro-6-oxo-pyridazinyl-3-oxy)-6-cyan-3-chromanol ("A"), F. 203-206°.

Analog erhält man aus den entsprechenden Epoxiden die nachstehenden 2,2-Dimethyl-4-(1-phenyl-1,6-dihydro-6-oxo-pyridazinyl-3-oxy)-3-chromanole:
6-Nitro-
6-Fluor-
6-Chlor-
6-Brom-
6-Trifluormethyl-
6-Methoxycarbonyl-
6-Ethoxycarbonyl-.

### Beispiel 2

Ein Gemisch von 1 g 2,2-Dimethyl-4-(6-oxo-1,6-dihydropyridazinyl-3-oxy)-6-cyan-3-chromanol ("IVa"), 2 ml Allylbromid, 3 g Kaliumcarbonat und 50 ml Aceton wird 2 Std. gekocht; danach werden weitere 2 ml Allylbromid zugefügt, und das Gemisch wird weitere 2 Std. gekocht. Man dampft ein, arbeitet wie üblich auf und erhält 2,2-Dimethyl-4-(1-allyl-1,6-dihydro-6-oxo-pyridazinyl-3-oxy)-6-cyan-3-chromanol, F. 145-146°.

Analog erhält man die nachstehenden 2,2-Dimethyl-6-cyan-3-chromanole:
4-(1-Propargyl-1,6-dihydro-6-oxo-pyridazinyl-3-oxy)-, F. 185-186°
4-[1-(2-Methoxyethyl)-1,6-dihydro-6-oxo-pyridazinyl-3-oxy]-
4-[1-(2-Ethoxyethyl)-1,6-dihydro-6-oxo-pyridazinyl-3-oxy]-
4-[1-(2-Formylethyl)-1,6-dihydro-6-oxo-pyridazinyl-3-oxy]-
4-[1-(2-Acetylethyl)-1,6-dihydro-6-oxo-pyridazinyl-3-oxy]-
4-[1-(2-Thioacetylethyl)-1,6-dihydro-6-oxo-pyridazinyl-3-oxy]-
4-(1-Carboxymethyl-1,6-dihydro-6-oxo-pyridazinyl-3-oxy)-F. 212-216°
4-(1-Methoxycarbonylmethyl-1,6-dihydro-6-oxo-pyridazinyl-3-oxy)-, F. 170-172°
4-(1-Ethoxycarbonylmethyl-1,6-dihydro-6-oxo-pyridazinyl-3-oxy)-
4-(1-Benzyloxycarbonylmethyl-1,6-dihydro-6-oxo-pyridazinyl-3-oxy)-, F. 162-164°
4-[1-(2-Nitroethyl)-1,6-dihydro-6-oxo-pyridazinyl-3-oxy]-
4-[1-( 2-Dimethylaminoethyl)-1,6-dihydro-6-oxo-pyridazinyl-3-oxy]-
4-(1-Cyanmethyl-1,6-dihydro-6-oxo-pyridazinyl-3-oxy)-, F. 201-203°
4-[1-(2-Cyanethyl)-1,6-dihydro-6-oxo-pyridazinyl-3-oxy]-
4-[1-(2-Fluorethyl)-1,6-dihydro-6-oxo-pyridazinyl-3-oxy]-
4-[1-(2,2,2-Trifluorethyl)-1,6-dihydro-6-oxo-pyridazinyl-3-oxy]-
4-[1-(2-Methylthio-ethyl)-1,6-dihydro-6-oxo-pyridazinyl-3-oxy]-
4-[1-(2-Methylsulfinyl-ethyl)-1,6-dihydro-6-oxo-pyridazinyl-3-oxy]-
4-[1-(2-Methylsulfonyl-ethyl)-1,6-dihydro-6-oxo-pyridazinyl-3-oxy]-
4-(1-Benzyl-1,6-dihydro-6-oxo-pyridazinyl-3-oxy)-, F. 197-199°.

Weiterhin erhält man analog die nachstehenden 2,2,3-Trimethyl-6-cyan-3-chromanole:
4-(1-Allyl-1,6-dihydro-6-oxo-pyridazinyl-3-oxy)-
4-(1-Propargyl-1,6-dihydro-6-oxo-pyridazinyl-3-oxy)-
4-[1-(2-Methoxyethyl)-1,6-dihydro-6-oxo-pyridazinyl-3-oxy]-
4-[1-(2-Ethoxyethyl)-1,6-dihydro-6-oxo-pyridazinyl-3-oxy]-
4-[1-(2-Formylethyl)-1,6-dihydro-6-oxo-pyridazinyl-3-oxy]-
4-[1-(2-Acetylethyl)-1,6-dihydro-6-oxo-pyridazinyl-3-oxy]-
4-[1-(2-Thioacetylethyl)-1,6-dihydro-6-oxo-pyridazinyl-3-oxy]-
4-(1-Carboxymethyl-1,6-dihydro-6-oxo-pyridazinyl-3-oxy)-
4-(1-Methoxycarbonylmethyl-1,6-dihydro-6-oxo-pyridazinyl-3-oxy)-
4-(1-Ethoxycarbonylmethyl-1,6-dihydro-6-oxo-pyridazinyl-3-oxy)-
4-(1-Benzyloxycarbonylmethyl-1,6-dihydro-6-oxo-pyridazinyl-3-oxy)-
4-[1-(2-Nitroethyl)-1,6-dihydro-6-oxo-pyridazinyl-3-oxy]-
4-[1-( 2-Dimethylaminoethyl)-1,6-dihydro-6-oxo-pyridazinyl-3-oxy]-
4-(1-Cyanmethyl-1,6-dihydro-6-oxo-pyridazinyl-3-oxy)-
4-[1-(2-Cyanethyl)-1,6-dihydro-6-oxo-pyridazinyl-3-oxy]-
4-[1-(2-Fluorethyl)-1,6-dihydro-6-oxo-pyridazinyl-3-oxy]-
4-[1-(2,2,2-Trifluorethyl)-1,6-dihydro-6-oxo-pyridazinyl-3-oxy]-
4-[1-(2-Methylthio-ethyl)-1,6-dihydro-6-oxo-pyridazinyl-3-oxy]-
4-[1-(2-Methylsulfinyl-ethyl)-1,6-dihydro-6-oxo-pyridazinyl-3-oxy]-
4-[1-(2-Methylsulfonyl-ethyl)-1,6-dihydro-6-oxo-pyridazinyl-3-oxy]-
4-(1-Benzyl-1,6-dihydro-6-oxo-pyridazinyl-3-oxy)-;
die nachstehenden 2,2-Dimethyl-6-nitro-3-chromanole:
4-(1-Allyl-1,6-dihydro-6-oxo-pyridazinyl-3-oxy)-
4-(1-Propargyl-1,6-dihydro-6-oxo-pyridazinyl-3-oxy)-
4-[1-(2-Methoxyethyl)-1,6-dihydro-6-oxo-pyridazinyl-3-oxy]-
4-[1-(2-Ethoxyethyl)-1,6-dihydro-6-oxo-pyridazinyl-3-oxy]-
4-[1-(2-Formylethyl)-1,6-dihydro-6-oxo-pyridazinyl-3-oxy]-
4-[1-(2-Acetylethyl)-1,6-dihydro-6-oxo-pyridazinyl-3-oxy]-
4-[1-(2-Thioacetylethyl)-1,6-dihydro-6-oxo-pyridazinyl-3-oxy]-
4-(1-Carboxymethyl-1,6-dihydro-6-oxo-pyridazinyl-3-oxy)-
4-(1-Methoxycarbonylmethyl-1,6-dihydro-6-oxo-pyridazinyl-3-oxy)-
4-(1-Ethoxycarbonylmethyl-1,6-dihydro-6-oxo-pyridazinyl-3-oxy)-
4-(1-Benzyloxycarbonylmethyl-1,6-dihydro-6-oxo-pyridazinyl-3-oxy)-
4-[1-(2-Nitroethyl)-1,6-dihydro-6-oxo-pyridazinyl-3-oxy]-
4-[1-(2-Dimethylaminoethyl)-1,6-dihydro-6-oxo-pyridazinyl-3-oxy]-
4-(1-Cyanmethyl-1,6-dihydro-6-oxo-pyridazinyl-3-oxy)-
4-[1-(2-Cyanethyl)-1,6-dihydro-6-oxo-pyridazinyl-3-oxy]-
4-[1-(2-Fluorethyl)-1,6-dihydro-6-oxo-pyridazinyl-3-oxy]-
4-[1-(2,2,2-Trifluorethyl)-1,6-dihydro-6-oxo-pyridazinyl-3-oxy]-
4-[1-(2-Methylthio-ethyl)-1,6-dihydro-6-oxo-pyridazinyl-3-oxy]-
4-[1-(2-Methylsulfinyl-ethyl)-1,6-dihydro-6-oxo-pyridazinyl-3-oxy]-
4-[1-(2-Methylsulfonyl-ethyl)-1,6-dihydro-6-oxo-pyridazinyl-3-oxy]-
4-(1-Benzyl-1,6-dihydro-6-oxo-pyridazinyl-3-oxy)-;
die nachstehenden 2,2-Dimethyl-6-brom-3-chromanole:
4-(1-Allyl-1,6-dihydro-6-oxo-pyridazinyl-3-oxy)-
4-(1-Propargyl-1,6-dihydro-6-oxo-pyridazinyl-3-oxy)-
4-[1-(2-Methoxyethyl)-1,6-dihydro-6-oxo-pyridazinyl-3-oxy]-
4-[1-(2-Ethoxyethyl)-1,6-dihydro-6-oxo-pyridazinyl-3-oxy]-
4-[1-(2-Formylethyl)-1,6-dihydro-6-oxo-pyridazinyl-3-oxy]-
4-[1-(2-Acetylethyl)-1,6-dihydro-6-oxo-pyridazinyl-3-oxy]-
4-[1-(2-Thioacetylethyl)-1,6-dihydro-6-oxo-pyridazinyl-3-oxy]-
4-(1-Carboxymethyl-1,6-dihydro-6-oxo-pyridazinyl-3-oxy)-
4-(1-Methoxycarbonylmethyl-1,6-dihydro-6-oxo-pyridazinyl-3-oxy)-
4-(1-Ethoxycarbonylmethyl-1,6-dihydro-6-oxo-pyridazinyl-3-oxy)-
4-(1-Benzyloxycarbonylmethyl-1,6-dihydro-6-oxo-pyridazinyl-3-oxy)-
4-[1-(2-Nitroethyl)-1,6-dihydro-6-oxo-pyridazinyl-3-oxy]-
4-[1-( 2-Dimethylaminoethyl)-1,6-dihydro-6-oxo-pyridazinyl-3-oxy]-
4-(1-Cyanmethyl-1,6-dihydro-6-oxo-pyridazinyl-3-oxy)-
4-[1-(2-Cyanethyl)-1,6-dihydro-6-oxo-pyridazinyl-3-oxy]-
4-[1-(2-Fluorethyl)-1,6-dihydro-6-oxo-pyridazinyl-3-oxy]-
4-[1-(2,2,2-Trifluorethyl)-1,6-dihydro-6-oxo-pyridazinyl-3-oxy]-
4-[1-(2-Methylthio-ethyl)-1,6-dihydro-6-oxo-pyridazinyl-3-oxy]-
4-[1-(2-Methylsulfinyl-ethyl)-1,6-dihydro-6-oxo-pyridazinyl-3-oxy]-
4-[1-(2-Methylsulfonyl-ethyl)-1,6-dihydro-6-oxo-pyridazinyl-3-oxy]-
4-(1-Benzyl-1,6-dihydro-6-oxo-pyridazinyl-3-oxy)-;
die nachstehenden 2,2-Dimethyl-6-methoxycarbonyl-3-chromanole:
4-(1-Allyl-1,6-dihydro-6-oxo-pyridazinyl-3-oxy)-
4-(1-Propargyl-1,6-dihydro-6-oxo-pyridazinyl-3-oxy)-
4-[1-(2-Methoxyethyl)-1,6-dihydro-6-oxo-pyridazinyl-3-oxy]-
4-[1-(2-Ethoxyethyl)-1,6-dihydro-6-oxo-pyridazinyl-3-oxy]-
4-[1-(2-Formylethyl)-1,6-dihydro-6-oxo-pyridazinyl-3-oxy]-
4-[1-(2-Acetylethyl)-1,6-dihydro-6-oxo-pyridazinyl-3-oxy]-
4-[1-(2-Thioacetylethyl)-1,6-dihydro-6-oxo-pyridazinyl-3-oxy]-
4-(1-Carboxymethyl-1,6-dihydro-6-oxo-pyridazinyl-3-oxy)-
4-(1-Methoxycarbonylmethyl-1,6-dihydro-6-oxo-pyridazinyl-3-oxy)-
4-(1-Ethoxycarbonylmethyl-1,6-dihydro-6-oxo-pyridazinyl-3-oxy)-
4-(1-Benzyloxycarbonylmethyl-1,6-dihydro-6-oxo-pyridazinyl-3-oxy)-
4-[1-(2-Nitroethyl)-1,6-dihydro-6-oxo-pyridazinyl-3-oxy]-
4-[1-(2-Dimethylaminoethyl)-1,6-dihydro-6-oxo-pyridazinyl-3-oxy]-
4-(1-Cyanmethyl-1,6-dihydro-6-oxo-pyridazinyl-3-oxy)-
4-[1-(2-Cyanethyl)-1,6-dihydro-6-oxo-pyridazinyl-3-oxy]-
4-[1-2-Fluorethyl)-1,6-dihydro-6-oxo-pyridazinyl-3-oxy)-
4-[1-(2,2,2-Trifluorethyl)-1,6-dihydro-6-oxo-pyridazinyl-3-oxy]-
4-[1-(2-Methylthio-ethyl)-1,6-dihydro-6-oxo-pyridazinyl-3-oxy]-
4-[1-(2-Methylsulfinyl-ethyl)-1,6-dihydro-6-oxo-pyridazinyl-3-oxy]-
4-[1-(2-Methylsulfonyl-ethyl)-1,6-dihydro-6-oxo-pyridazinyl-3-oxy]-
4-(1-Benzyl-1,6-dihydro-6-oxo-pyridazinyl-3-oxy)-;
sowie
2,2-Tetramethylen-4-(1-allyl-1,6-dihydro-6-oxo-pyridazinyl-3-oxy)-6-cyan-3-chromanol
2,2-Pentamethylen-4-(1-allyl-1,6-dihydro-6-oxo-pyridazinyl-3-oxy)-6-cyan-3-chromanol.

### Beispiel 3

Ein Gemisch von 1 g IVa, 0,67 ml Aziridin und 10 ml Dioxan wird im Bombenrohr 3 Std. auf 130° erhitzt. Man dampft ein, löst den Rückstand in verdünnter Salzsäure, wäscht mit Dichlormethan, macht alkalisch und arbeitet wie üblich auf. Das erhaltene 2,2-Dimethyl-4-[1-(2-aminoethyl)-1,6-dihydro-6-oxo-pyridazinyl-3-oxy]-6-cyan-3-chromanol wird aus Acetonitril/Diethylether 1:1 umkristallisiert. F. 167-169°.

Analog erhält man die folgenden 2,2-Dimethyl-4-[1-(2-aminoethyl)-1,6-dihydro-6-oxo-pyridazinyl-3-oxy]-3-chromanole:
6-Nitro-
6-Fluor-
6-Chlor-
6-Brom-
6-Trifluormethyl-
6-Methoxycarbonyl-
6-Ethoxycarbonyl-.

### Beispiel 4

In ein Gemisch von 1 g IVa, 1,6 g K₂CO₃ und 35 ml Aceton wird unter Rühren und Kochen 8 Std. Ethylenoxid eingeleitet. Man läßt über Nacht stehen, filtriert, dampft das Filtrat ein und erhält nach üblicher Aufarbeitung 2,2-Dimethyl-4-[1-(2-hydroxyethyl)-1,6-dihydro-6-oxo-pyridazinyl-3-oxy]-6-cyan-3-chromanol, F. 152-154°.

Analog erhält man die folgenden 2,2-Dimethyl-4-[1-(2-hydroxyethyl)-1,6-dihydro-6-oxo-pyridazinyl-3-oxy]-3-chromanole:
6-Nitro-
6-Fluor-
6-Chlor-
6-Brom-
6-Trifluormethyl-
6-Methoxycarbonyl-
6-Ethoxycarbonyl-.

### Beispiel 5

Eine Lösung von 2,82 g 2,2-Dimethyl-4-brom-6-cyan-3-chromanol und 2,5 g 3-Hydroxy-1-phenyl-1,6-dihydro-pyridazin-6-on in 70 ml DMSO wird mit 1,2 g 80%igem NaH versetzt und 3 Tage bei 20° gerührt. Nach üblicher Aufarbeitung erhält man "A", F. 203-206°.

### Beispiel 6

Ein Gemisch von 1 g "A", 1,5 g dl-Campher-10-sulfonsäurechlorid und 15 ml Pyridin wird 1,5 Std. bei 90° gerührt. Man dampft ein, löst den Rückstand in Ethylacetat, wäscht mit verdünnter Salzsäure und mit Wasser, trocknet die organische Phase über Na₂SO₄, dampft erneut ein, löst in Dichlormethan und reinigt chromatographisch an Kieselgel. Man erhält 1,1 g eines Gemisches diastereomerer 4-Camphersulfonsäureester, das in 45 ml Methanol gelöst wird. Man gibt 6 g NaOH auf Träger (E. Merck, Art.Nr. 1567) hinzu, rührt 16 Std. bei 20°, dampft ein, arbeitet wie üblich auf und erhält 2,2-Dimethyl-4-(1-phenyl-1,6-dihydro-6-oxo-pyridazinyl-3-oxy)-6-cyan-3-chromen neben wenig "A" (chromatographisch abgetrennt).

### Beispiel 7

Eine Lösung von 1 g 2,2-Dimethyl-4-(1-benzyloxycarbonyl-methyl-1,6-dihydro-6-oxo-pyridazinyl-3-oxy)-6-cyan-3-chromanol in 15 ml Methanol wird an 0,3 g 5%ig. Pd-C bei 20° und 1 bar bis zum Stillstand hydriert. Man filtriert, dampft ein und erhält 2,2-Dimethyl-4-(1-carboxymethyl-1,6-dihydro-6-oxo-pyridazinyl-3-oxy)-6-cyan-3-chromanol, F. 212-216°.

### Beispiel 8

Ein Gemisch von 2 g "A", 11,7 ml Ameisensäure und 3,3 ml Acetanhydrid wird 16 Std. bei 20° stehengelassen und anschließend 2 Std. auf 40-42° erwärmt. Nach Eindampfen und üblicher Aufarbeitung erhält man 2,2-Dimethyl-3-formyloxy-4-(1-phenyl-1,6-dihydro-6-oxo-pyridazinyl-3-oxy)-6-cyan-chroman.

Analog erhält man aus den entsprechenden 3-Hydroxychromanen:
2,2,3-Trimethyl-3-formyloxy-4-(1-phenyl-1,6-dihydro-6-oxo-pyridazinyl-3-oxy)-6-cyan-chroman
2,2-Dimethyl-3-formyloxy-4-(1-allyl-1,6-dihydro-6-oxo-pyridazinyl-3-oxy)-6-cyan-chroman
2,2,3-Trimethyl-3-formyloxy-4-(1-allyl-1,6-dihydro-6-oxo-pyridazinyl-3-oxy)-6-cyan-chroman.

### Beispiel 9

Ein Gemisch von 1 g "A" und 5 ml Acetanhydrid wird 1 Std. gekocht. Man kühlt ab, arbeitet wie üblich auf und erhält 2,2-Dimethyl-3-acetoxy-4-(1-phenyl-1,6-dihydro-6-oxo-pyridazinyl-3-oxy)-6-cyan-chroman.

Analog erhält man:
2,2,3-Trimethyl-3-acetoxy-4-(1-phenyl-1,6-dihydro-6-oxo-pyridazinyl-3-oxy)-6-cyan-chroman
2,2-Dimethyl-3-acetoxy-4-(1-allyl-1,6-dihydro-6-oxo-pyridazinyl-3-oxy)-6-cyan-chroman
2,2,3-Trimethyl-3-acetoxy-4-(1-allyl-1,6-dihydro-6-oxo-pyridazinyl-3-oxy)-6-cyan-chroman.

### Beispiel 10

Eine Lösung von 1 g 2,2-Dimethyl-4-(1-phenyl-1,6-dihydro-6-oxo-pyridazinyl-3-oxy)-6-nitro-3-chromanol in 25 ml Methanol wird bei 20° und 1 bar an 0,5 g 5%igem Pd-C bis zum Stillstand hydriert. Man filtriert, dampft ein und erhält 2,2-Dimethyl-4-(1-phenyl-1,6-dihydro-6-oxo-pyridazinyl-3-oxy)-6-amino-3-chromanol.

Analog erhält man:
2,2,3-Trimethyl-4-(1-phenyl-1,6-dihydro-6-oxo-pyridazinyl-3-oxy)-6-amino-3-chromanol.

### Beispiel 11

Eine Lösung von 1 g 2,2-Dimethyl-4-(1-phenyl-1,6-dihydro-6-oxo-pyridazinyl-3-oxy)-6-amino-3-chromanol in 15 ml HCOOH und 1 ml Pyridin wird 19 Std. gekocht und eingedampft. Nach üblicher Aufarbeitung erhält man 2,2-Dimethyl-4-(1-phenyl-1,6-dihydro-6-oxo-pyridazinyl-3-oxy)-6-formamido-3-chromanol.

### Beispiel 12

Ein Gemisch von 1 g 2,2-Dimethyl-4-(1-phenyl-1,6-dihydro-6-oxo-pyridazinyl-3-oxy)-6-amino-3-chromanol, 10 ml Acetanhydrid und 10 ml Pyridin wird 16 Std. bei 20° stehengelassen. Man dampft ein, reinigt chromatographisch und erhält 2,2-Dimethyl-4-(1-phenyl-1,6-dihydro-6-oxo-pyridazinyl-3-oxy)-6-acetamido-3-chromanol.

### Beispiel 13

In eine siedende Lösung von 1 g "A" in 50 ml Methanol und 2 ml Wasser wird 14 Std. unter Rühren HCl eingeleitet. Man läßt erkalten und über Nacht stehen. Die ausgefallene 2,2-Dimethyl-4-(1-phenyl-1,6-dihydro-6-oxo-pyridazinyl-3-oxy)-3-chromanol-6-carbonsäure wird abfiltriert.

### Beispiel 14

Ein Gemisch von 3 g "A", 31 g Na₃PO₄.12 H₂O, 28 ml Pyridin, 28 ml Wasser, 67 ml Essigsäure und 25 g Raney-Ni (wasserfeucht) wird bei 20° 3 Std. gerührt. Nach Filtration arbeitet man wie üblich auf und erhält 2,2-Dimethyl-4-(1-phenyl-1,6-dihydro-6-oxo-pyridazinyl-3-oxy)-6-formyl-3-chromanol, F. 256-257°.

### Beispiel 15

Man löst 3 g "A" in 40 ml tert.-Butanol und gibt unter Rühren 5,6 g gepulvertes KOH hinzu. Nach 1 Std. Kochen und üblicher Aufarbeitung erhält man 2,2-Dimethyl-4-(1-phenyl-1,6-dihydro-6-oxo-pyridazinyl-3-oxy)-6-carbamoyl-3-chromanol.

### Beispiel 16

In eine Lösung von 3 g "A" in einem Gemisch von 20 ml Pyridin und 10 ml Triethylamin leitet man 5 Std. bei 20° H₂S ein, dampft ein, arbeitet wie üblich auf und erhält 2,2-Dimethyl-4-(1-phenyl-1,6-dihydro-6-oxo-pyridazinyl-3-oxy)-6-thiocarbamoyl-3-chromanol.

Die nachstehenden Beispiele betreffen pharmazeutische Zubereitungen, welche Verbindungen der Formel I und/oder deren physiologisch unbedenkliche Salze enthalten.

### Beispiel A Tabletten

Ein Gemisch von 1 g 2,2-Dimethyl-4-(1-allyl-1,6-dihydro-6-oxo-pyridazinyl-3-oxy)-6-cyan-3-chromanol, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 0,1 mg Wirkstoff enthält.

### Beispiel B Dragees

Analog Beispiel A werden Tabletten gepreßt, die anschliessend in üblicher Weise mit einem Überzug aus Saccharose, Kartoffelstärke, Talk, Tragant und Farbstoff überzogen werden.

### Beispiel C Kapseln

Man füllt 1 kg 2,2,3-Trimethyl-4-(1-allyl-1,6-dihydro-6-oxo-pyridazinyl-3-oxy)-6-cyan-3-chromanol in üblicher Weise in Hartgelatinekapseln, so daß jede Kapsel 0,5 mg Wirkstoff enthält.

### Beispiel D Ampullen

Eine Lösung von 10 g Na-Salz von 2,2-Dimethyl-4-(1-carboxymethyl-1,6-dihydro-6-oxo-pyridazinyl-3-oxy)-6-cyan-3-chromanol in 70 l 1,2-Propandiol wird mit zweifach destilliertem Wasser auf 100 l aufgefüllt, steril filtriert, in Ampullen abgefüllt und steril verschlossen. Jede Ampulle enthält 0,1 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

## Patentansprüche (Patentansprüche für folgende(n) Vertragsstaat(en): AT, BE, CH, LI, DE, DK, FR, GB, GR, IT, LU, NL, SE)

1. Chromanderivate der Formel I 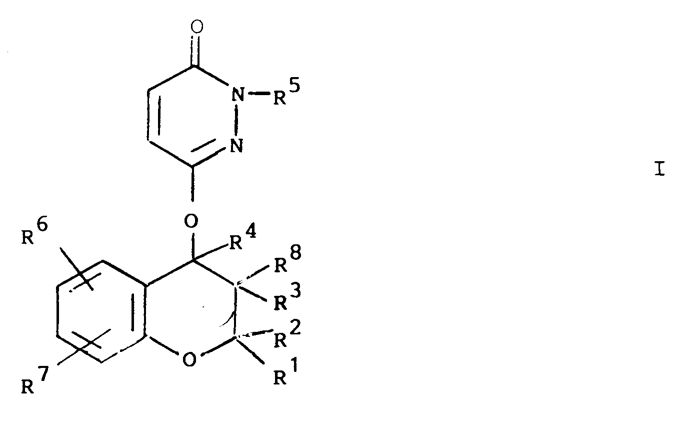 worin
R¹ A,
R² und R⁸ jeweils H oder A,
R¹ und R² zusammen auch Alkylen mit 3-6 C-Atomen,
R³ OH, OA oder OAc,
R⁴ H,
R³ und R⁴ zusammen auch eine Bindung,
R⁵ Ar oder CₙH₂ₙ-R⁹,

2. 2,2-Dimethyl-4-(1-allyl-1,6-dihydro-6-oxo-pyridazinyl-3-oxy)-6-cyan-3-chromanol gemäß Anspruch 1.
R⁹ Alkenyl oder Alkinyl mit jeweils 2-4 C-Atomen, OH, OA, CHO, CO-A, CS-A, COOH, COOA, COO-alkyl-Ar, CS-OA, NO₂, NH₂, NHA, NA₂, CN, F, Cl, Br, J, CF₃, SA, SO-A, SO₂-A oder Ar,
Ar eine unsubstituierte oder eine ein- oder zweifach durch R¹⁰ substituierte Phenylgruppe,
R⁶, R⁷ und R¹⁰ jeweils H, A, HO, AO, CHO, ACO, CF₃CO, ACS, HOOC, AOOC, AO-CS, ACOO, A-CS-O, Hydroxy-alkyl, Mercapto-alkyl, NO₂, NH₂, NHA, NA₂, CN, F, Cl, Br, J, CF₃, AS, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitro-alkyl, Cyan-alkyl, A-C(=NOH), A-C(=NNH₂), H₂PO₃ oder A₂PO₃,
n 1, 2 oder 3,
A Alkyl mit 1-6 C-Atomen,
-alkyl Alkylen mit 1-6 C-Atomen und
Ac Alkanoyl mit 1-8 C-Atomen oder Aroyl mit 7-11 C-Atomen
bedeuten,
sowie deren Salze.

3. Verfahren zur Herstellung von Chromanderivaten der Formel I gemäß Anspruch 1, dadurch gekennzeichnet, daß man ein Chroman der Formel II 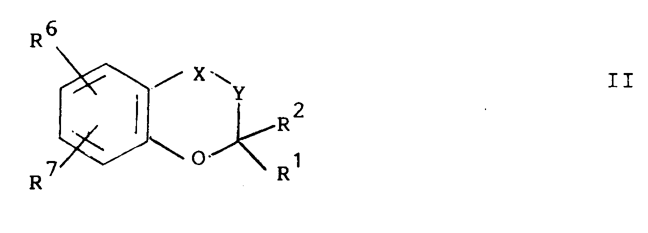 worin
X-Y oder -CHE-CR³R⁸- und 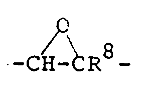
E Cl, Br, J oder eine reaktionsfähig veresterte OH-Gruppe bedeuten und
R¹, R², R³, R⁶, R⁷ und R⁸ die bei Formel I angegebenen Bedeutungen haben,
mit einer Verbindung der Formel III 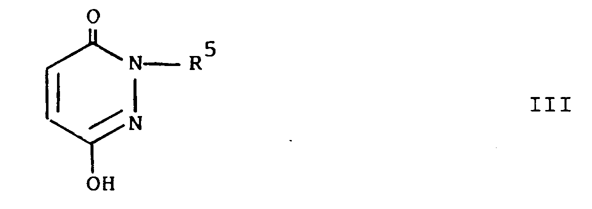 worin R⁵ die bei Formel I angegebene Bedeutung hat
oder mit einem ihrer reaktionsfähigen Derivate umsetzt
oder daß man eine Verbindung der Formel IV 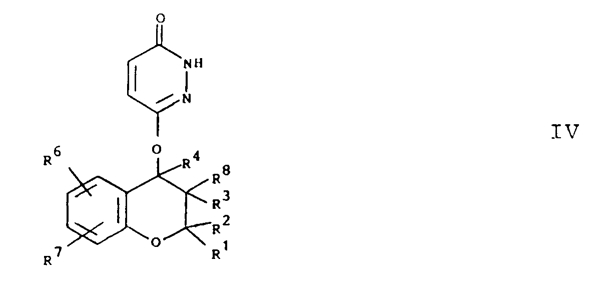 worin R¹, R², R³, R⁴, R⁶, R⁷ und R⁸ die bei Formel I angegebenen Bedeutungen haben,
oder eines ihrer reaktionsfähigen Derivate
mit einer Verbindung der Formel
E-R⁵ V
worin R⁵ und E die bei Formel I bzw. bei Formel II angegebenen Bedeutungen haben
oder einem ihrer reaktionsfähigen Derivate
umsetzt
und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶ und/oder R⁷ in andere Reste R³, R⁵, R⁶ und/oder R⁷ umwandelt und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I zur Bekämpfung von Krankheiten.

7. Verwendung von Verbindungen der Formel I zur Herstellung eines Arzneimittels.

## Patentansprüche (Patentansprüche für folgende(n) Vertragsstaat(en): ES)

1. Verfahren zur Herstellung von Chromanderivaten der Formel I 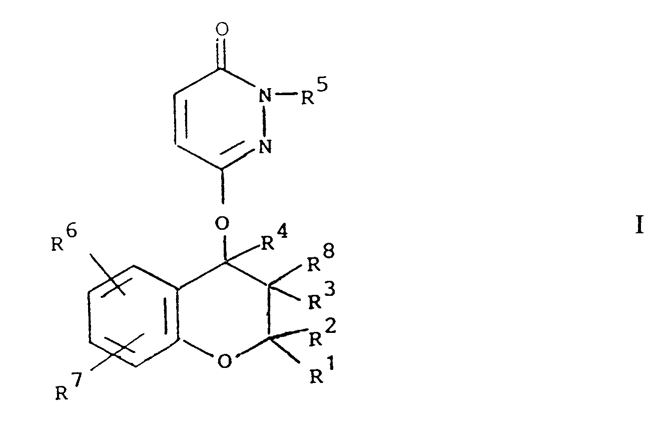 worin
R¹ A,
R² und R⁸ jeweils H oder A,
R¹ und R² zusammen auch Alkylen mit 3-6 Atomen,
R³ OH, OA oder OAc,
R⁴ H,
R³ und R⁴ zusammen auch eine Bindung,
R⁵ Ar oder CₙH₂ₙ-R⁹,
R⁹ Alkenyl oder Alkinyl mit jeweils 2-4 C-Atomen, OH, OA, CHO, CO-A, CS-A, COOH, COOA, COO-alkyl-Ar, CS-OA, NO₂, NH₂, NHA, NA₂, CN, F, Cl, Br, J, CF₃, SA, SO-A, SO₂-A oder Ar,
Ar eine unsubstituierte oder eine ein- oder zweifach durch R¹⁰ substituierte Phenylgruppe,
R⁶, R⁷ und R¹⁰ jeweils H, A, HO, AO, CHO, ACO, CF₃CO, ACS, HOOC, AOOC, AO-CS, ACOO, A-CS-O, Hydroxy-alkyl, Mercapto-alkyl, NO₂, NH₂, NHA, NA₂, CN, F, Cl, Br, J, CF₃, AS, ASO, ASO₂, AO-SO, AO-SO₂, AcNH, AO-CO-NH, H₂NSO, HANSO, A₂NSO, H₂NSO, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitrol-alkyl, Cyan-alkyl, A-C(=NOH), A-C(=NNH₂), H₂PO₃ oder A₂PO₃,
n 1, 2 oder 3,
A Alkyl mit 1-6 C-Atomen,
-alkyl Alkylen mit 1-6 C-Atomen und
Ac Alkanoyl mit 1-8 C-Atomen oder Aroyl mit 7-11 C-Atomen
bedeuten,
sowie deren Salze, dadurch gekennzeichnet, daß man ein Chroman der Formel II 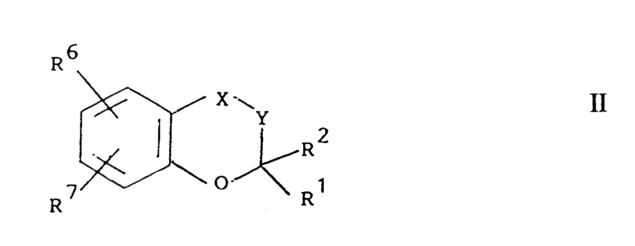 worin
X-Y 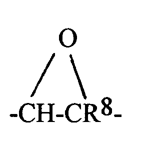 oder -CHE-CR³R⁸ - und
E Cl, Br, J oder eine reaktionsfähig veresterte OH-Gruppe bedeuten und
R¹, R², R³, R⁶, R⁷ und R⁸ die bei Formel I angegebenen Bedeutungen haben,
mit einer Verbindung der Formel III 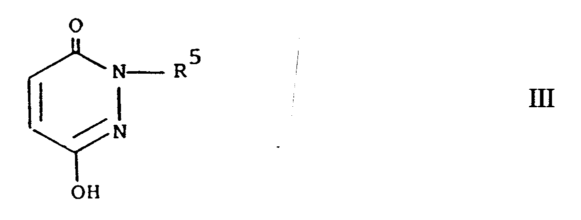 worin R5 die bei Formel I angegebene Bedeutung hat oder mit einem ihrer reaktionsfähigen Derivate umsetzt
oder daß man eine Verbindung der Formel IV 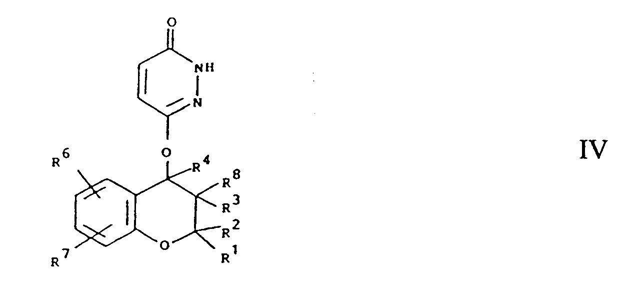 worin R¹, R², R³, R⁴, R⁶, R⁷ und R⁸ die bei Formel I angegebene Bedeutungen haben,
oder eines ihrer reaktionsfähigen Derivate
mit einer Verbindung der Formel
E-R⁵ V
worin R⁵ und E die bei Formel I bzw. bei Formel II angegebenen Bedeutungen haben
oder einem ihrer reaktionsfähigen Derivate
umsetzt
und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶ und/oder R⁷ in andere Reste R³, R⁵, R⁶ und/oder R⁷ umwandelt und/oder daß man eine basische Verbindung der Formel I durch Behandeln mit einer Säure in eines ihrer Säureadditionssalze umwandelt.

2. Verfahren zur Herstellung von 2,2-Dimethyl-4-(1-allyl-1,6-dihydro-6-oxopyridazinyl-3-oxy)-6-cyan-3-chromanol gemäß Anspruch 1.

3. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

## Claims (Claims for the following Contracting State(s): AT, BE, CH, LI, DE, DK, FR, GB, GR, IT, LU, NL, SE)

1. Chroman derivatives of the formula I 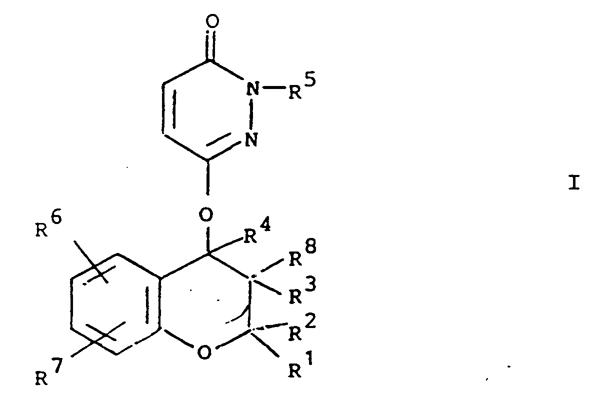 in which
R¹ is A,
R² and R⁸ are each H or A,
R¹ and R² together are also alkylene with 3-6 C atoms,
R³ is OH, OA or OAc,
R⁴ is H,
R³ and R⁴ together are also a bond,
R⁵ is Ar or CₙH₂ₙ-R⁹,
R⁹ is alkenyl or alkynyl with 2-4 C atoms in each case, OH, OA, CHO, CO-A, CS-A, COOH, COOA, COO-alkyl-Ar, CS-OA, NO₂, NH₂, NHA, NA₂, CN, F, Cl, Br, I, CF₃, SA, SO-A, SO₂-A or Ar,
Ar is a phenyl group which is unsubstituted or substituted once or twice by R¹⁰,
R⁶, R⁷ and R¹⁰ are each H, A, HO, AO, CHO, ACO, CF₃CO, ACS, HOOC, AOOC, AO-CS, ACOO, A-CS-O, hydroxy-alkyl, mercapto-alkyl, NO₂, NH₂, NHA, NA₂, CN, F, Cl, Br, I, CF₃, AS, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, nitro-alkyl, cyano-alkyl, A-C(=NOH), A-C(=NNH₂), H₂PO₃ or A₂PO₃,
n is 1, 2 or 3,
A is alkyl with 1-6 C atoms,
-alkyl is alkylene with 1-6 C atoms and
Ac is alkanoyl with 1-8 C atoms or aroyl with 7-11 C atoms,
and the salts thereof.

2. 2,2-Dimethyl-4-(1-allyl-1,6-dihydro-6-oxo-pyridazinyl-3-oxy)-6-cyano-3-chromanol according to Claim 1.

3. Process for the preparation of chroman derivatives of the formula I according to Claim 1, characterized in that a chroman of the formula II 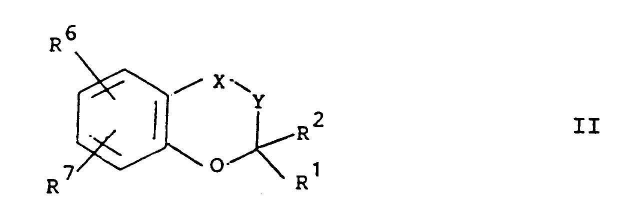 in which
X-Y is 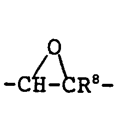 or -CHE-CR³R⁸- and
E is Cl, Br, I or a reactive esterified OH group, and
R¹, R², R³, R⁶, R⁷ and R⁸ have the meanings indicated for formula I, is reacted with a compound of the formula III 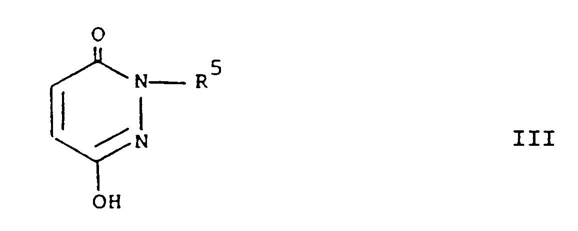 in which R⁵ has the meaning indicated for formula I, or with one of the reactive derivatives thereof, or in that a compound of the formula IV 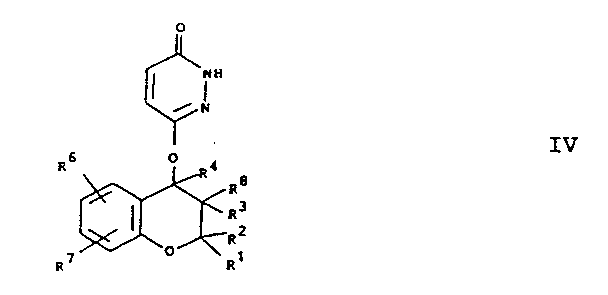 in which R¹, R², R³, R⁴, R⁶, R⁷ and R⁸ have the meanings indicated for formula I, or one of the reactive derivatives thereof, is reacted with a compound of the formula
E-R⁵ V
in which R⁵ and E have the meanings indicated for formula I and for formula II respectively, or one of the reactive derivatives thereof,
and/or in that a compound of the formula I in which R³ is OH and R⁴ is H is dehydrated and/or in that one or more of the radicals R³, R⁵, R⁶ and/or R⁷ in a compound of the formula I are converted into other radicals R³, R⁵, R⁶ and/or R⁷ and/or in that a basic compound of the formula I is converted into one of its acid addition salts by treatment with an acid.

4. Process for the preparation of pharmaceutical formulations, characterized in that a compound of the formula I and/or one of its physiologically acceptable salts is converted together with at least one solid, liquid or semiliquid vehicle or auxiliary into a suitable dosage form.

5. Pharmaceutical formulation characterized by containing at least one compound of the formula I and/or one of the physiologically acceptable salts thereof.

6. Compounds of the formula I for controlling diseases.

7. Use of compounds of the formula I for the preparation of a medicament.

## Claims (Claims for the following Contracting State(s): ES)

1. Process for the preparation of chroman derivatives of the formula I 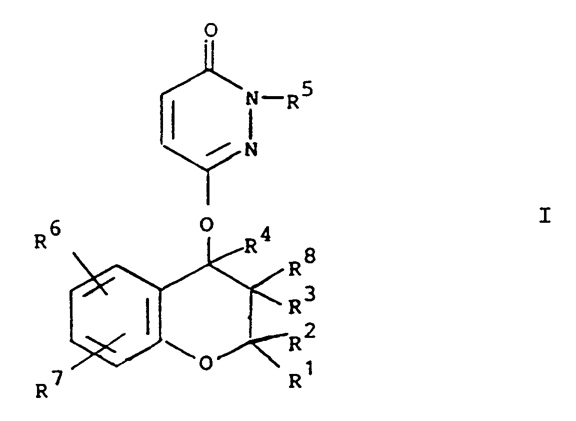 in which
R¹ is A,
R² and R⁸ are each H or A,
R¹ and R² together are also alkylene with 3-6 C atoms,
R³ is OH, OA or OAc,
R⁴ is H,
R³ and R⁴ together are also a bond,
R⁵ is Ar or CₙH₂ₙ-R⁹,
R⁹ is alkenyl or alkynyl with 2-4 C atoms in each case, OH, OA, CHO, CO-A, CS-A, COOH, COOA, COO-alkyl-Ar, CS-OA, NO₂, NH₂, NHA, NA₂, CN, F, Cl, Br, I, CF₃, SA, SO-A, SO₂-A or Ar,
Ar is a phenyl group which is unsubstituted or substituted once or twice by R¹⁰,
R⁶, R⁷ and R¹⁰ are each H, A, HO, AO, CHO, ACO, CF₃CO, ACS, HOOC, AOOC, AO-CS, ACOO, A-CS-O, hydroxy-alkyl, mercapto-alkyl, NO₂, NH₂, NHA, NA₂, CN, F, Cl, Br, I, CF₃, AS, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, nitro-alkyl, cyano-alkyl, A-C(=NOH), A-C(=NNH₂), H₂PO₃ or A₂PO₃,
n is 1, 2 or 3,
A is alkyl with 1-6 C atoms,
-alkyl is alkylene with 1-6 C atoms and
Ac is alkanoyl with 1-8 C atoms or aroyl with 7-11 C atoms,
and the salts thereof, characterized in that a chroman of the formula II 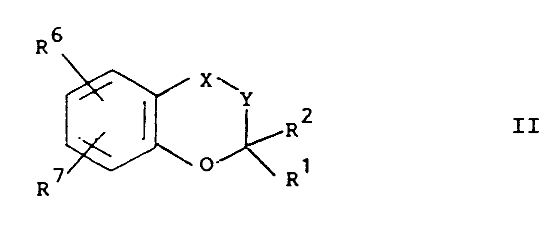 in which
X-Y is 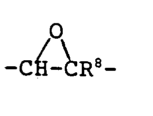 or -CHE-CR³R⁸- and
E is Cl, Br, I or a reactive esterified OH group, and
R¹, R², R³, R⁶, R⁷ and R⁸ have the meanings indicated for formula I, is reacted with a compound of the formula III 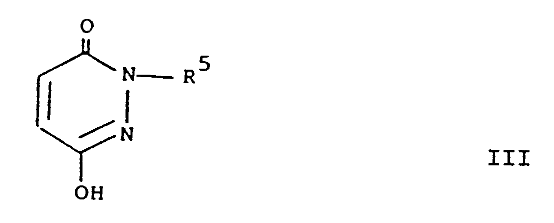 in which R⁵ has the meaning indicated for formula I, or with one of the reactive derivatives thereof, or in that a compound of the formula IV 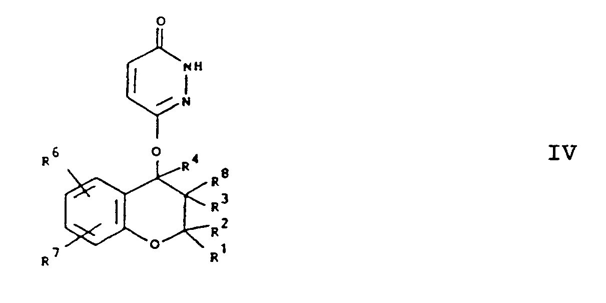 in which R¹, R², R³, R⁴, R⁶, R⁷ and R⁸ have the meanings indicated for formula I, or one of the reactive derivatives thereof, is reacted with a compound of the formula
E-R⁵ V
in which R⁵ and E have the meanings indicated for formula I and for formula II respectively, or one of the reactive derivatives thereof,
and/or in that a compound of the formula I in which R³ is OH and R⁴ is H is dehydrated and/or in that one or more of the radicals R³, R⁵, R⁶ and/or R⁷ in a compound of the formula I are converted into other radicals R³, R⁵, R⁶ and/or R⁷ and/or in that a basic compound of the formula I is converted into one of its acid addition salts by treatment with an acid.

2. Process for the preparation of 2,2-dimethyl-4-(1-allyl-1,6-dihydro-6-oxo-pyridazinyl-3-oxy)-6-cyano--3-chromanol according to claim 1.

3. Process for the preparation of pharmaceutical formulations, characterized in that a compound of the formula I and/or one of its physiologically acceptable salts is converted together with at least one solid, liquid or semiliquid vehicle or auxiliary into a suitable dosage form.

## Revendications (Revendications pour l'(les) Etat(s) contractant(s) suivant(s): AT, BE, CH, LI, DE, DK, FR, GB, GR, IT, LU, NL, SE)

1. Dérivés du chromane de formule I 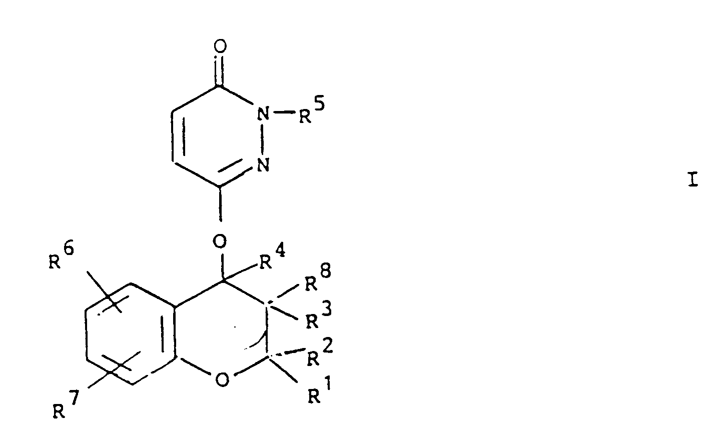 où
R¹ représente A,
R² et R⁸ représentent chaque fois H ou A,
R¹ et R² représentent également ensemble un alkylène ayant de 3 à 6 atomes de C,
R³ représente OH, OA ou OAc,
R⁴ représente H,
R³ et R⁴ représentent aussi ensemble une liaison,
R⁵ représente Ar ou CₙH₂ₙ-R⁹,
R⁹ représente un alcényle ou un alcynyle ayant chaque fois de 2 à 4 atomes de C, OH, OA, CHO, CO-A, CS-A, COOH, COOA, COO-alkyl-Ar, CS-OA, NO₂, NH₂, NHA, NA₂, CN, F, Cl, Br, I, CF₃, SA, SO-A, SO₂-A ou Ar,
Ar représente un groupe phényle non substitué ou substitué une ou deux fois par R¹⁰,
R⁶, R⁷ et R¹⁰ représentent chaque fois H, A, HO, AO, CHO, ACO, CF₃CO, ACS, HOOC, AOOC, AO-CS, ACOO, A-CS-O, un hydroxy-alkyle, un mercapto-alkyle, NO₂, NH₂, NHA, NA₂, CN, F, Cl, Br, I, CF₃, AS, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, un ACO-alkyle, un nitro-alkyle, un cyano-alkyle, A-C(=NOH), A-C(=NNH₂), H₂PO₃ ou A₂PO₃,
n représente 1, 2 ou 3,
A représente un alkyle ayant de 1 à 6 atomes de C,
-alkyle représente un alkylène ayant de 1 à 6 atomes de C et
Ac représente un alcanoyle ayant de 1 à 8 atomes de C ou un aroyle avec de 7 à 11 atomes de C
ainsi que leurs sels.

2. 2,2-diméthyl-4-(1-allyl-1,6-dihydro-6-oxo-pyridazinyl-3-oxy)-6-cyano-3-chromanol selon la revendication 1.

3. Procédé pour préparer des dérivés du chromane de formule I selon la revendication 1, caractérisé en ce que l'on fait réagir un chromane de formule II 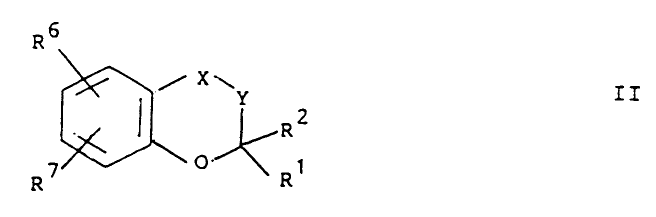 où R⁵ et E ont les significations données pour la formule I ou pour la formule II
ou avec un de ses dérivés susceptibles de réagir
et/ou en ce que l'on déshydrate un composé de formule I, où R³ représente OH et R⁴ représente H, et/ou en ce que, dans un composé de formule I, on transforme un ou plusieurs des radicaux R³, R⁵, R⁶ et/ou R⁷ en d'autres radicaux R³, R⁵, R⁶ et/ou R⁷ et/ou en ce que l'on transforme un composé basique de formule I, par traitement avec un acide, en un de ses sels d'addition d'acide.

4. Procédé pour préparer des compositions pharmaceutiques, caractérisé en ce que l'on amène sous une forme appropriée de dosage un composé de formule I et/ou un de ses sels sans inconvénient physiologique avec au moins une matière support ou un additif solide, liquide ou semi-liquide.

5. Préparation pharmaceutique, caractérisée en ce qu'elle comporte au moins un composé de formule I et/ou un de ses sels sans inconvénient physiologique.

6. Composés de formule I pour lutter contre des maladies.

7. Utilisation des composés de formule I pour préparer un médicament.
où
X-Y représente 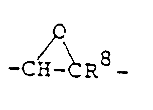 ou -CHE-CR³R⁸- et
E représente Cl, Br, I ou un groupe OH estérifié susceptible de réagir et
R¹, R², R³, R⁶, R⁷ et R⁸ ont les significations données pour la formule I,
avec un composé de formule III 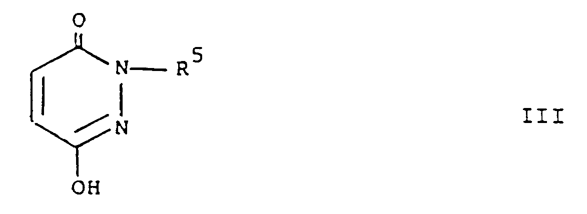 où R⁵ a la signification donnée pour la formule I
ou avec un de ses dérivés susceptible de réagir
ou en ce qu'on fait réagir un composé de formule IV 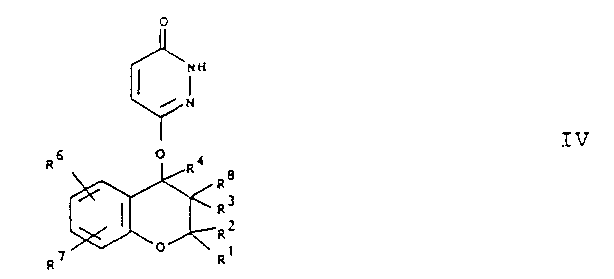 où R¹, R², R³, R⁴, R⁶, R⁷ et R⁸ ont les significations données pour la formule I,
ou un de ses dérivés susceptibles de réagir
avec un composé de formule
E-R⁵ V

## Revendications (Revendications pour l'(les) Etat(s) contractant(s) suivant(s): ES)

1. Procédé pour préparer des dérivés du chromane de formule I 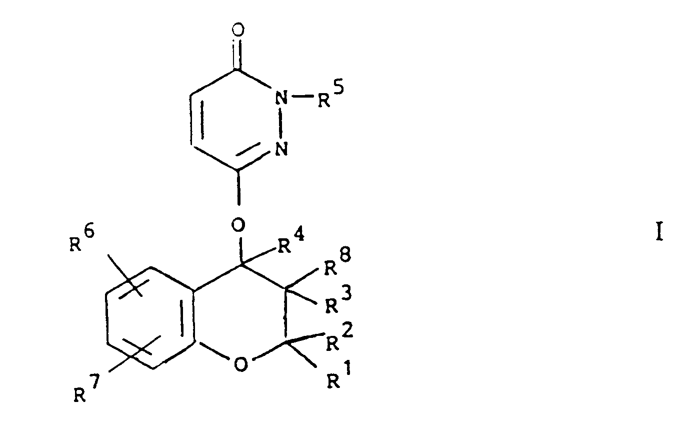 où
R¹ représente A,
R² et R⁸ représentent chaque fois H ou A,
R¹ et R² représentent également ensemble un alkylène ayant de 3 à 6 atomes de C,
R³ représente OH, OA ou OAc,
R⁴ représente H,
R³ et R⁴ représentent aussi ensemble une liaison,
R⁵ représente Ar ou CₙH₂ₙ-R⁹,
R⁹ représente un alcényle ou un alcynyle ayant chaque fois de 2 à 4 atomes de C, OH, OA, CHO, CO-A, CS-A, COOH, COOA, COO-alkyl-Ar. CS-OA, NO₂, NH₂, NHA, NA₂, CN, F, Cl, Br, I, CF₃, SA, SO-A, SO₂-A ou Ar,
Ar représente un groupe phényle non substitué ou substitué une ou deux fois par R¹⁰,
R⁶, R⁷ et R¹⁰ représentent chaque fois H, A, HO, AO, CHO, ACO, CF₃CO, ACS, HOOC, AOOC, AO-CS, ACOO, A-CS-O, un hydroxy-alkyle, un mercapto-alkyle, NO₂, NH₂, NHA, NA₂, CN, F, Cl, Br, I, CF₃, AS, ASO, ASO₂, AO-SO, AO-SO₂, AcNH, AO-CO-NH, H₂NSO, HANSO, A₂NSO, H₂NSO, HANSO₂, A₂NSO₂, H₂NCO, HANCO, A₂NCO, H₂NCS, HANCS, A₂NCS, ASONH, ASO₂NH, AOSONH, AOSO₂NH, un ACO-alkyle, un nitro-alkyle, un cyano-alkyle, A-C(=NOH), A-C(=NNH₂), H₂PO₃ ou A₂PO₃,
n représente 1, 2 ou 3,
A représente un alkyle ayant de 1 à 6 atomes de C,
-alkyle représente un alkylène ayant de 1 à 6 atomes de C et
Ac représente un alcanoyle ayant de 1 à 8 atomes de C ou un aroyle avec de 7 à 11 atomes de C
ainsi que leurs sels,
caractérisé en ce que l'on fait réagir un chromane de formule II 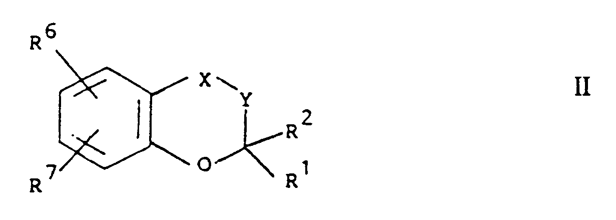 où
X-Y représente 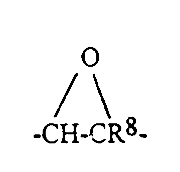 ou -CHE-CR³R⁸- et
E représente Cl, Br, I ou un groupe OH estérifié susceptible de réagir et
R¹, R², R³, R⁶, R⁷ et R⁸ ont les significations données pour la formule I,
avec un composé de formule III 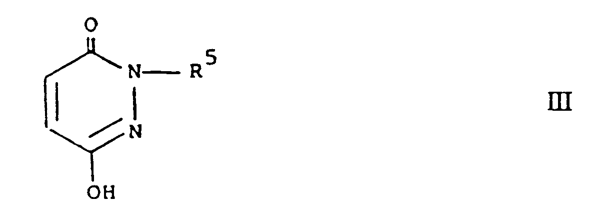 où R⁵ a la signification donnée pour la formule I
ou avec un de ses dérivés susceptible de réagir
ou en ce qu'on fait réagir un composé de formule IV 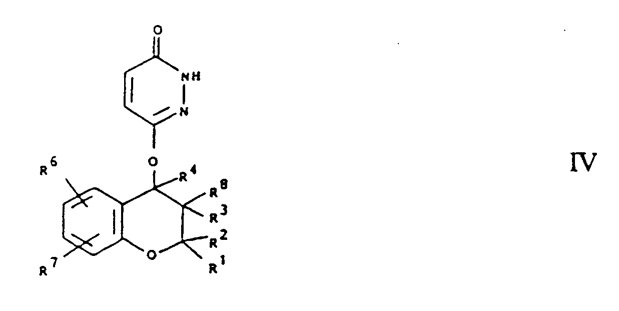 où R¹, R², R³, R⁴, R⁶, R⁷ et R⁸ ont les significations données pour la formule I,
ou un de ses dérivés susceptibles de réagir
avec un composé de formule
E-R⁵ V
où R⁵ et E ont les significations données pour la formule I ou pour la formule II
ou avec un de ses dérivés susceptibles de réagir
et/ou en ce que l'on déshydrate un composé de formule I, où R³ représente OH et R⁴ représente H, et/ou en ce que, dans un composé de formule I, on transforme un ou plusieurs des radicaux R³, R⁵, R⁶ et/ou R⁷ en d'autres radicaux R³, R⁵, R⁶ et/ou R⁷ et/ou en ce que l'on transforme un composé basique de formule I, par traitement avec un acide, en un de ses sels d'addition d'acide.

2. Procédé pour préparer le 2,2-diméthyl-4-(1-allyl-1,6-dihydro-6-oxo-pyridazinyl-3-oxy)-6-cyano-3-chromanol selon la revendication 1.

3. Procédé pour préparer des compositions pharmaceutiques, caractérisé en ce que l'on amène sous une forme appropriée de dosage un composé de formule I et/ou un de ses sels sans inconvénient physiologique avec au moins une matière support ou un additif solide, liquide ou semi-liquide.
